Europäisches Patentamt

(19) European Patent Office

Office européen des brevets

(11) Publication number: **0 274 226 B1**

(12) **EUROPEAN PATENT SPECIFICATION**

(45) Date of publication of patent specification:
13.03.91 Bulletin 91/11

(51) Int. Cl.⁵: **C07C 31/22, C07C 29/38**

(21) Application number: 87310718.9

(22) Date of filing: 04.12.87

(54) **Process for the production of glycerol from formaldehyde.**

(30) Priority: 10.12.86 GB 8629494

(43) Date of publication of application:
13.07.88 Bulletin 88/28

(45) Publication of the grant of the patent:
13.03.91 Bulletin 91/11

(84) Designated Contracting States:
BE DE FR GB IT NL

(56) References cited:
GB-A- 509 484
GB-A- 740 398
GB-A- 2 106 511
THE JOURNAL OF THE AMERICAN CHEMICAL SOCIETY, vol. 106, no. 17, 22nd August 1984, pages 4829-4832, American Chemical Society, Columbus, Ohio, US; T. MATSUMOTO et al.: "Selective formation of triose from formaldehyde catalyzed by thiazolium salt"

(73) Proprietor: BP Chemicals Limited
Belgrave House 76 Buckingham Palace Road
London, SW1W 0SU (GB)

(72) Inventor: Gracey, Benjamin Patrick
c/o BP Chemicals Limited, Salt End
Hull, HU12 8DS (GB)
Inventor: Hudson, Barry
c/o BP Chemicals Limited, Salt End
Hull, HU12 8DS (GB)
Inventor: Williams, Peter Sefton
c/o BP Chemicals Limited, Salt End
Hull, HU12 8DS (GB)

(74) Representative: Krishnan, Suryanarayana Kalyana et al
BP INTERNATIONAL LIMITED Patents & Agreements Division Chertsey Road
Sunbury-on-Thames Middlesex TW16 7LN (GB)

## Description

The present invention relates to a process for the synthesis of glycerol from formaldehyde.

Glycerol is a valuable raw material for the production of various esters, printing inks, foodstuffs, in antifreezes, as a moistening agent in tobacco and in soaps and for producing nitroglycerine.

Hitherto glycerol has been produced from animal and vegetable oils and fats, in which it occurs as the glyceryl ester of mainly palmitic, stearic and oleic acids, by hydrolysis or hydrogenolysis. Glycerol is also obtained in large quantities as a by-product in the manufacture of soap, and this is still a commercial source of glycerol. Another method of preparing glycerol is the fermentation of glucose to which sodium sulphite has been added (the yield is 20-25%) Glycerol can also be produced from propylene which is converted to allyl chloride, thereafter into allyl alcohol, then into a monochlorohydrin which is finally hydrolysed into glycerol.

A new process is to add osmium tetroxide and hydrogen peroxide to acraldehyde ; this produces glyceraldehyde which is then catalytically hydrogenated to glycerol.

Thus, apart from two relatively expensive and complicated synthetic routes, the primary commercial source of glycerol is still as a by-product from soap and fatty alcohol manufacture.

Matsumoto, T. et al in Journal of the American Chemical Society, 1984, 106, pp 4829-4832 describe a method of synthesising dihydroxy acetone from formaldehyde (as paraformaldehyde) catalysed by 3-ethylbenzothiazolium bromide in the presence of triethylamine in ethanol at 100°C. ICI's USP 4024193 describes, amongst others, hydrogenation of dihydroxy acetone to glycerol using a homogeneous catalyst system comprising a ruthenium triphenyl phosphine complex and a strong acid. The source of the dihydroxyacetone used as feedstock is not disclosed.

We have now surprisingly found that glycerol can be produced from formaldehyde in a single step in a liquid reaction medium.

Accordingly, the present invention provides a process for the production of glycerol from formaldehyde which process comprises reacting at elevated temperature formaldehyde in a liquid reaction medium with hydrogen in the presence of a catalyst system comprising as essential components (i) a thiazolium or imidazolium salt, (ii) a proton abstractor, and (iii) a compound or mixture containing (a) a rhodium or ruthenium moiety and optionally (b) a moiety of the formula $XR_3$ wherein X is either phosphorus, nitrogen, arsenic or antimony and the groups R are independently either hydrogen or hydrocarbyl or substituted hydrocarbyl groups.

The process of the present invention offers advantages over the prior art synthetic route in that it is relatively simple and economical.

Formaldehyde may be added in monomeric, oligomeric or polymeric form. In monomeric form it may be added either as formaldehyde gas or as a solution of formaldehyde in an organic solvent, suitably an alkanol, for example methanol, ethanol or propanol, or a mixture thereof. A suitable solution of formaldehyde in an organic solvent is that conventionally referred to as "alcoform". In oligomeric form, formaldehyde may be added as trioxan. In polymeric form, formaldehyde may be added in the form of the solid, paraformaldehyde.

Hydrogen is readily available on a commercial scale. It may be used in a commercially available form or may, if desired, be further purified. The hydrogen partial pressure may suitably be in the range from 0.1 to 300 bar absolute, preferably from 1 to 50 bar absolute.

Hydrogen containing other gases inert under the reaction conditions, for example carbon monoxide, may also be used in the process of the invention. Although carbon monoxide may not have a chemical effect on the reaction, it may have a physical effect related to its contribution to the total pressure of gases in the system which in turn may affect the product distribution. Other inert gases, for example methane, may also be present.

The component (i) in the catalyst system may be an aliphatic, aromatic or a heterocyclic thiazolium salt or an imidazolium salt. Specific examples of such salts include the halides, especially the bromide and iodide salts. Of these the 3-methyl benzothiazolium iodide, 3-ethylbenzothiazolium bromide, 3-isopropyl benzothiazolium bromide, 3-ethyl thiazolium bromide, thiamine thiazolium chloride and 1-methyl 3-ethylbenzimidazolium bromide are specific examples. A salt bound to a polymeric backbone or oxide support may be used in order to facilitate separation thereof from the reaction products and to facilitate regeneration and thereby reuse of the catalyst.

The proton abstractor (ii) in the catalyst may suitably be an amine, which may be primary, secondary, or tertiary and can be aliphatic, alicyclic, aromatic or heterocyclic or a phosphine, which may be an alkyl or aryl phosphine or a mixture of alkyl/aryl phosphines or such proton abstractors when supported on or immobilized by, for example, an ion-exchange resin or silica. Specific examples of the amines include triethylamine, imidazole, pyridine, pyrimidine, piperazine, amidine- or guanidine-type bases. Specific examples of the phosphines include triphenyl phosphine and triethylphosphine.

The component (iii) of the catalyst is a compound or mixture containing a rhodium or ruthenium moiety (a) and optionally a moiety (b) of the formula $XR_3$ wherein X is either phosphorus, nitrogen, arsenic or antimony and the groups R are independently either hydrogen or hydrocarbyl or substituted hydrocarbyl groups. Preferably a moiety (b) of the formula $XR_3$ is employed. Preferably X in the formula is phosphorus. The group R in the formula is preferably a hydrocarbyl or substituted hydrocarbyl group. Suitable hydrocarbyl groups include alkyl groups, cycloalkyl groups and aryl groups, which may be substituted or unsubstituted. The component (iii) may suitably combine the moieties (a) and (b) in a single compound, for example as the compound $RhCl(PPh_3)_3$ or the compound $Ru(H)(OAc)(PPh_3)_3$. Alternatively, the moieties (a) and (b) may be added in the form of separate compounds, for example as $RuCl_3$ and $PPh_3$. It will be appreciated that the moiety (b) and the proton abstractor may be identical.

The component (iii) of the catalyst, particularly when this takes the form of a single compound, may be supported on a suitable support. Suitable supports include organic polymers, for example polystyrene, inorganic oxide materials, and carbons.

The relative molar ratios of formaldehyde to the catalyst components may vary over a moderately wide range.

Again, the molar ratio of formaldehyde to one of the catalyst components (i) or (ii) may vary over a wide range. In a typical catalyst system comprising 3-ethyl benzothiazolium bromide and triethylamine in equimolar proportions, the molar ratio of formaldehyde to the 3-ethyl benzothiazolium bromide can vary, for example from 5 : 1 to 500 : 1.

The liquid medium is suitably a solvent capable of dissolving both formaldehyde and, when a non-supported catalyst is used, the catalyst system. Specific examples of the solvents that can be used include the aliphatic alcohols, e.g. ethanol, n-propanol, iso-propanol and the butanols ; esters such as ethyl acetate and ethyl propionate ; dioxan ; dimethyl sulphoxide ; dimethylformamide ; and mixtures thereof.

The process is operated at an elevated temperature, which may suitably be in the range from 25 to 250°C, preferably from 50 to 150°C.

The process may be operated batchwise or continuously, preferably continuously.

The advantages associated with single step operation, as compared with two-step operation, are self-evident. The principal advantage is the saving in capital cost of equipment arising from operating the process in a single reactor and avoidance of the requirement for separation apparatus, with associated savings in operating costs.

The process of the invention will now be further illustrated by reference to the following Examples.

## Example 1

To a Fischer Porter vessel was charged paraformaldehyde (1.8 g), 3-ethylbenzothiazolium bromide (0.73 g), triethylamine (0.3 g), dimethylformamide (11.8 g) and $RuCl_2(PPh_3)_3$ (0.5 g). The vessel was purged and pressurised to 100 psig with hydrogen and then heated with stirring to 100°C for 10 hours. Analysis of the product mixture by gas liquid chromatography indicated essentially complete conversion of the formaldehyde to 7% dihydroxyacetone and 22% glycerol, with the balance being almost entirely methanol.

## Example 2

To a Fischer Porter vessel was charged paraformaldehyde (1.8 g), 3-ethylbenzothiazolium bromide (0.73 g), 1, 5, 7 triazobycyclo [4, 4, 0] dec-5-ene (0.42 g), dimethylformamide (11.8 g) and $RuCl_2(PPh_3)_3$ (0.5 g). The vessel was purged and pressurised to 100 psig with hydrogen and heated with stirring to 100°C for 10 hours. Analysis of the product mixture by gas liquid chromotography indicated essentially complete conversion of the formaldehyde to 3% dihydroxyacetone and 8.2% glycerol, with the balance being nearly all methanol.

## Example 3

To a Fischer-Porter vessel was charged paraformaldehyde (1.8 g) and methanol (12.5 ml). The vessel was purged and pressurised to 30 psig with nitrogen and then heated to 130°C for 15 mins to predissolve the formaldehyde. After cooling, 3-ethylbenzothiazolium bromide (0.73 g), triethylamine (0.3 g) and $RuCl_2(PPh_3)_3$ (0.5 g) was added to the mixture. The vessel was again purged and then pressurised to 100 psig with hydrogen. It was heated with stirring to 100°C for 3 hrs. Analysis of the product mixture by HPLC and g.c. showed 0.2% conversion to dihydroxyacetone and 37% conversion to glycerol.

## Example 4

To a Fischer-Porter vessel was charged paraformaldehyde (1.8 g) and methanol (12.5 ml). The vessel was purged and pressurised to 30 psig with nitrogen and then heated to 130°C for 15 mins to predissolve the formaldehyde. After cooling, 3-ethylbenzothiazolium bromide (0.73 g), triethylamine (0.3 g) and $Ru_3(CO)_{12}$ (0.5 g) was added. The vessel was again purged and then pressurised with 100 psig of hydrogen and 20 psig CO. It was heated with stirring to 100°C for 3 hours. Analysis of the product mixture hy HPLC and g.c. showed 1.4% conversion to dihydroxyacetone and 18% conversion to glycerol.

## Claims

1. A process for the production of glycerol from formaldehyde which process comprises reacting at elevated temperature formaldehyde in a liquid reaction medium with hydrogen in the presence of a catalyst system comprising as esse ʼal components (i) a thiazolium or imidazolium salt, (ii) a proton abstractor, and (iii) a compound or mixture containing (a) a rhodium or ruthenium moiety and optionally (b) a moiety of the formula $XR_3$ wherein X is either phosphorus, nitrogen, arsenic or antimony and the groups R are independently either hydrogen or hydrocarbyl or substituted hydrocarbyl groups.

2. A process according to claim 1 wherein the formaldehyde is added either as formaldehyde gas or as a solution of formaldehyde in an organic solvent.

3. A process according to claim 1 wherein the formaldehyde is added in the form of paraformaldehyde.

4. A process according to any one of the preceding claims wherein the hydrogen partial pressure is in the range from 1 to 50 bar.

5. A process according to any one of the preceding claims wherein as the component (i) there is used a bromide or iodide.

6. A process according to claim 5 wherein component (i) is either 3-methyl benzothiazolium iodide, 3-ethyl benzothiazolium bromide, 3-isopropyl benzothiazolium bromide, 3-ethyl thiazolium bromide or thiamine thiazolium chloride or 1-methyl 3-ethylbenzimidazolium bromide.

7. A process according to either claim 5 or claim 6 wherein the thiazolium or imidazolium salt (i) is bound to a polymeric backbone or oxide support.

8. A process according to any one of the preceding claims wherein the proton abstractor is either an amine or a phosphine.

9. A process according to claim 8 wherein the proton abstractor is an amine which is either triethylamine, imidazole, pyridine, pyrimidine, piperazine or a strong amidine or guanidine-type base.

10. A process according to claim 8 wherein the proton abstractor is a phosphine which is either triphenylphosphine or triethylphosphine.

11. A process according to any one of the preceding claims wherein there is employed a moiety (b) of the formula $XR_3$ wherein X is phosphorus.

12. A process according to any one of the preceding claims wherein there is employed a moiety (b) of the formula $XR_3$ wherein R is either an alkyl, cycloalkyl or aryl group.

13. A process according to either claim 11 or claim 12 wherein component (iii) combines the moieties (a) and (b) in a single compound.

14. A process according to either claim 11 or claim 12 wherein the moieties (a) and (b) of component (iii) are in the form of separate compounds.

15. A process according to any one of the preceding claims wherein the liquid medium is either an aliphatic alcohol, an ester, dioxan, dimethyl sulphoxide or dimethylformamide.

16. A process according to any one of the preceding claims wherein the elevated temperature is in the range from 25 to 250°C.

17. A process according to any one of claims 1 to 15 wherein the elevated temperature is in the range from 50 to 150°C.

## Ansprüche

1. Verfahren zur Herstellung von Glycerin aus Formaldehyd, wobei das Verfahren die Umsetzung von Formaldehyd mit Wasserstoff bei erhöhter Temperatur und in einem flüssigen Reaktionsmedium umfaßt, in Gegenwart eines Katalysatorsystems, das als wesentliche Komponenten umfaßt (i) ein Thiazolium- oder Imidazoliumsalz, (ii) einen Protonenentzieher und (iii) eine Verbindung oder Mischung enthaltend (a) einen Rhodium- oder Rutheniumanteil und wahlweise (b) einen Anteil mit der Formel $XR_3$, worin X entweder Phosphor, Stickstoff, Arsen oder Antimon ist und die Gruppen R unabhängig entweder Wasserstoff oder Hydrocarbyl- oder substituierte Hydrocarbylgruppen sind.

2. Verfahren nach Anspruch 1, worin das Formaldehyd entweder als Formaldehydgas zugegeben wird oder als Lösung von Formaldehyd in einem organischen Lösungsmittel.

3. Verfahren nach Anspruch 1, worin das Formaldehyd in Form von Paraformaldehyd zugegeben wird.

4. Verfahren nach einem der vorstehenden Ansprüche, worin der Wasserstoff-Partialdruck im Bereich von 1 bis 50 bar ist.

5. Verfahren nach einem der vorstehenden Ansprüche, worin als Komponente (i) ein Bromid oder Iodid verwendet wird.

6. Verfahren nach Anspruch 5, worin Komponente (i) entweder 3-Methyl-benzothiazoliumiodid, 3-Ethyl-benzothiazoliumbromid, 3-Isopropyl-benzothiazoliumbromid3-Ethyl-thiazoli umbromid oder Thiamin-thiazoliumchlorid oder 1-Methyl-3-ethylbenzimidazoliumbromid ist.

7. Verfahren nach Anspruch 5 oder Anspruch 6, worin das Thiazolium- oder Imidazoliumsaiz (i) an ein polymeres Gerüst oder einen Oxidträger gebunden ist.

8. Verfahren nach einem der vorstehenden Ansprüche, worin der Protonenentzieher entweder ein Amin oder ein Phosphin ist.

9. Verfahren nach Anspruch 8, worin der Protonenentzieher ein Amin ist, das entweder Triethylamin, Imidazol, Pyridin, Pyrimidin, Piperazin oder eine starke Base vom Amidin- oder Guanidin-Typ ist.

10. Verfahren nach Anspruch 8, worin der Protonenentzieher ein Phosphin ist, das entweder Triphenylphosphin oder Triethylphosphin ist.

11. Verfahren nach einem der vorstehenden Ansprüche, worin ein Anteil (b) mit der Formel $XR_3$

verwendet wird, worin X Phosphor ist.

12. Verfahren nach einem der vorstehenden Ansprüche, worin ein Anteil (b) mit der Formel $XR_3$ verwendet wird, worin R entweder eine Alkyl-, Cycloalkyl- oder Arylgruppe ist.

13. Verfahren nach Anspruch 11 oder Anspruch 12, worin Komponente (iii) die Anteile (a) und (b) in einer einzigen Verbindung vereint.

14. Verfahren nach Anspruh 11 oder Anspruch 12, worin die Anteile (a) und (b) der Komponente (iii) in Form von separaten Verbindungen sind.

15. Verfahren nach einem der vorstehenden Ansprüche, worin das flüssige Medium entweder ein aliphatischer Alkohol, ein Ester, Dioxan, Dimethylsulfoxid oder Dimethylformamid ist.

16. Verfahren nach einem der vorstehenden Ansprüche, worin die erhöhte Temperatur im Bereich von 25 bis 250°C ist.

17. Verfahren nach einem der Ansprüche 1 bis 15, worin die erhöhte Temperatur im Bereich von 50 bis 150°C ist.


## Revendications

1. Procédé de production de glycérol à partir de formaldéhyde, procédé qui comprend la réaction à température élevée de formaldéhyde, dans un milieu de réaction liquide, avec de l'hydrogène en présence d'un système de catalyseur comprenant comme composants essentiels (i) un sel de thiazolium ou d'imidazolium, (ii) un extracteur de protons, et (iii) un composé ou mélange contenant (a) une partie de rhodium ou de ruthéniun et, éventuellement, (b) une partie de la formule $XR_3$, dans laquelle X est soit le phosphore, l'azote, l'arsenic ou l'antimoine et les groupes R sont indépendamment soit l'hydrogène, soit un groupe hydrocarbyle, soit des groupes hydrocarbyle substitués.

2. Procédé selon la revendication 1, dans lequel le formaldéhyde est ajouté sous forme de gaz de formaldéhyde ou sous forme de solution de formaldéhyde dans un solvant organique.

3. Procédé selon la revendication 1, dans lequel le formaldéhyde est ajouté sous forme de paraformaldéhyde.

4. Procédé selon l'une des revendications précédentes, dans lequel la pression partielle d'hydrogène est de l'ordre de 1 à 50 bars.

5. Procédé selon l'une des revendications précédentes, dans lequel il est utilisé, comme composant (i), un bromure ou un iodure.

6. Procédé selon la revendication 5, dans lequel le composant (i) est soit de l'iodure de 3-méthyibenzothiazolium, du bromure de 3-éthylbenzothiazolium, du bromure de 3-isopropylbenzothiazolium, du bromure de 3-éthylthiazolium, soit du chlorure de thiamine thiazolium, soit du bromure de 1-méthyle 3-éthylbenzimidazolium.

7. Procédé selon les revendications 5 ou 6, dans lequel le sel de thiazolium ou d'imidazolium (i) est lié à une chaîne polymère ou un support oxyde.

8. Procédé selon l'une des revendications précédentes, dans lequel l'extracteur de protons est une amine ou une phosphine.

9. Procédé selon la revendication 8, dans lequel l'extracteur de protons est une amine qui est soit la triéthylamine, l'imidazole, la pyridine, la pyrimidine, la pipérazine, soit une base forte de type amidine ou guanidine.

10. Procédé selon la revendication 8, dans lequel l'extracteur de protons est une phosphine qui est soit la triphénylphosphine, soit la triéthylphosphine.

11. Procédé selon l'une des revendications précédentes, dans lequel il est utilisé une partie (b) de la formule $XR_3$, dans laquelle X est le phosphore.

12. Procédé selon l'une des revendications précédentes, dans lequel il est utilisé une partie (b) de la formule $XR_3$, dans laquelle R est un groupe alcoyle, cycloalcoyle ou aryle.

13. Procédé selon les revendications 11 ou 12, dans lequel le composant (iii) combine les parties (a) et (b) en un seul composé.

14. Procédé selon les revendications 11 ou 12, dans lequel les parties (a) et (b) du composant (iii) se présentent sous forme de composés séparés.

15. Procédé selon l'une des revendications précédentes, dans lequel le milieu liquide est un alcool aliphatique, un ester, le dioxane, le sulfoxyde de diméthyle ou le diméthylformamide.

16. Procédé selon l'une des revendications précédentes, dans lequel la température élevée est comprise entre 25 et 250°C.

17. Procédé selon l'une ou l'autres des revendications 1 à 15, dans lequel la température élevée est comprise entre 50 et 150°C.